(19) Europäisches Patentamt
European Patent Office
Office européen des brevets

(11) EP 4 289 288 A1

(12) EUROPEAN PATENT APPLICATION
published in accordance with Art. 153(4) EPC

(43) Date of publication:
13.12.2023  Bulletin 2023/50

(21) Application number: 22911535.7

(22) Date of filing: 03.10.2022

(51) International Patent Classification (IPC):
A23L 33/105 (2016.01)    A23L 29/00 (2016.01)
A23L 2/38 (2021.01)    C12N 1/20 (2006.01)
C12R 1/01 (2006.01)

(52) Cooperative Patent Classification (CPC):
A23L 2/38; A23L 29/00; A23L 33/105; C12N 1/20;
C12R 2001/01

(86) International application number:
PCT/KR2022/014864

(87) International publication number:
WO 2023/120896 (29.06.2023 Gazette 2023/26)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 24.12.2021  KR 20210187096
18.09.2022  KR 20220117527

(71) Applicant: Binotec Co., Ltd.
Daegu 42151 (KR)

(72) Inventors:
• KIM, Yu Mi
Daegu 42151 (KR)
• JANG, Gi Hyun
Daegu 42151 (KR)
• SEOK, Chang Hwan
Daegu 42151 (KR)
• SHIN, Ji Won
Daegu 42151 (KR)
• GWAK, Yun Geum Sang
Daegu 42151 (KR)

(74) Representative: Mewburn Ellis LLP
Aurora Building
Counterslip
Bristol BS1 6BX (GB)

(54) **NOVEL LACTOBACILLUS PLANTARUM BNT_G401 STRAIN AND USE THEREOF**

(57)    The objective of the present invention is to provide a novel Lactiplantibacillus plantarum BNT_G401 strain and uses thereof.

[Fig. 8]

EP 4 289 288 A1

**Description**

**Technical field**

The present invention relates to a novel Lactiplantibacillus Plantarum BNT_G401 strain and uses thereof.

**Background art**

$\gamma$-aminobutyric acid (GABA) is a non-proteinogenic amino acid which is known to have various physiological functions such as promoting brain function, suppressing blood pressure rise and diuretic action, reducing appetite, improving liver function, promoting alcohol metabolism, acting as a deodorant, as well as playing the role as an inhibitory neurotransmitter in the brain.

As the functionality of the GABA is getting known, GABA is gaining attention not only as a medicine but also as a functional food material. Currently, GABA is naturally contained in fruits, rice, green tea, cabbage etc., but the contents thereof are low, and thus it is difficult to expect physiological activities and efficacy thereof with the amount consumed from natural food alone.

Curcuma longa L., a plant belonging to Zingiberaceae, is a perennial plant, and grows naturally in hot and humid South Asia, Africa, and Central and South America. In Korea, as mass cultivation of Curcuma longa L. has been successful in Jindo Island, it has recently been cultivated all over the country. Curcuma longa L. contains a yellow pigment called Curcumin as its main ingredient, thereby exhibiting strong antioxidative activity, and the rhizome used as a medicinal part contains Curcumin the most. Since Curcuma longa L. has effects in improving liver function, it is known to promote liver cell regeneration and liver detoxification, and have choleretic effects, gastric juice secretion promoting effects, diuretic effects, anticancer, anti-inflammatory and antioxidative effects, etc.

Korean Patent Publication No. 10-2011-0106924 discloses a hepatoprotection fermented food comprising fermented Curcuma longa L. and a method of manufacturing the same.

Korean Patent Publication No. 10-2012-0066803 discloses a composition containing fermented Curcuma longa L. for the prevention and treatment of non-alcoholic fatty liver.

Korean Patent Publication No. 10-2016-0035454 discloses a novel bacterium lactobacillus brevis THK-734, and a method for producing GABA using the same.

Currently, Curcuma longa L. extracts are also utilized as an herbal composition for preventing and treating liver diseases. However, researches on hangover relief or weight loss effects of fermented Curcuma longa L. with increased GABA production using lactic acid strains have hardly been reported.

**Summary of invention**

**Technical task**

The present invention is to provide a novel strain with GABA producing ability.

The present invention is to provide a fermented product with high GABA production using the novel strain.

The present invention is to provide a composition useful for weight loss and hangover relief using the fermented product.

The present invention is to provide a method for manufacturing the fermented product.

**Means for solving technical task**

Embodiments of the present invention will be described in detail with reference to the accompanying drawings so that those having ordinary knowledge in the art to which the present invention pertains can easily carry out the invention. The present invention can be implemented in various different forms, and is not limited by the embodiments explained herein.

Throughout the specification, when a part "includes" a certain component, it means that other components may be further included rather than excluding other components, unless otherwise specified.

As used in the present invention, the term "about" means within 10%, preferably within 5%, and more preferably within 1% of a given value or range.

**Novel Lactiplantibacillus plantarum strain**

In an embodiment, the present invention provides a novel Lactiplantibacillus plantarum strain.

In the present invention, the Lactiplantibacillus plantarum may be Lactiplantibacillus plantarum BNT_G401 strain deposited as Accession No. KCCM 13179P.

The Lactiplantibacillus plantarum BNT_G401 strain was separated from fermented Kimchi in order to find a novel lactic acid bacterium capable of biosynthesizing γ-aminobutyric acid (GABA), and was deposited with the Korean Culture Center of Microorganisms (KCCM) on May 17, 2022 and given Accession No. KCCM 13179P. As the novel strain is a strain capable of biosynthesizing GABA with monosodium glutamate (MSG) as a substrate, it has GABA biosynthesis ability.

Accordingly, the present invention provides the use of the Lactiplantibacillus plantarum strain, particularly the Lactiplantibacillus plantarum BNT_G401 strain deposited as KCCM 13179P, in order to manufacture a fermented product for the production of high content of GABA. Additionally, the present invention provides a composition for manufacturing a fermented product for GABA biosynthesis comprising the strain.

## GABA containing fermented product

In an embodiment, the present invention provides a fermented product containing high content of GABA. The fermented product is produced by inoculating a medium composition with the Lactiplantibacillus plantarum strain and fermenting the same.

In a specific embodiment, the present invention provides a GABA containing fermented product, which is produced by inoculating a medium composition comprising: a Curcuma longa L. extract having a solid content of 0.5 to 10%; monosodium glutamate (MSG); a carbon source; and a nitrogen source with a Lactiplantibacillus plantarum strain and fermenting the same.

The term "fermented product" as used herein means a product of enzymatic or metabolic degradation using microorganisms. Specifically, the term may mean a product cultured after inoculating the medium composition comprising a Curcuma longa L. extract with a Lactiplantibacillus plantarum strain, particularly Lactiplantibacillus plantarum BNT_G401 strain deposited as KCCM 13179P. Additionally, the term "fermented product" may be interchangeably used with "fermentation," "Curcuma longa L. fermented product," etc. in the present application.

The term "medium" or "medium composition" as used herein means a substance in which nutrients required for culturing the microorganism or strain are mixed as main ingredients. The medium composition supplies nutrients and growth factors, etc. including water essential for survival and growth. Furthermore, the medium composition of the present application comprises optimal compositions and contents for producing GABA, and particularly comprises, as essential ingredients, a Curcuma longa L. extract, MSG, a carbon source, and a nitrogen source. Also, a phosphorous source, an inorganic compound, an amino acid and/or vitamin, etc. may be further included. The medium composition of the present invention may be cultured by adjusting the temperature, pH, etc. under aerobic conditions in an ordinary medium.

The medium composition may properly comprise, as essential ingredients, a Curcuma longa L. extract, MSG, a carbon source, and a nitrogen source in an amount generally used in the pertinent art.

The Curcuma longa L. extract used in the present application may be an extract extracted by treating dried Curcuma longa L. with extraction solvents such as water or ethanol, etc. In this case, the final solid content of Curcuma longa L. in the Curcuma longa L. extract may be 0.5 to 10%, specifically 1 to 7%, more specifically 1 to 5%, and particularly 1, 3, or 5%.

The term "extract" as used herein includes a crude extract obtained by treating with extraction solvents such as water or ethanol, etc. as well as a processed product of an extract. For example, the Curcuma longa L. extract may be manufactured in a powder state by additional processes such as vacuum distillation and freeze drying or spray drying, etc.

In a specific example, the medium composition may include about 10 to 50% by weight, specifically about 10 to 30% by weight, more specifically about 15 to 25% by weight, and particularly about 20% by weight of Curcuma longa L. extract with respect to a total weight of the medium composition.

The monosodium glutamate (MSG) used in the present application is used as a biosynthesis substrate of GABA. Specifically, GABA is produced as a result of the conversion of glutamate by glutamate decarboxylase (GAD). GAD is a pyridoxal 5'-phosphate (PLP)-dependent intracellular enzyme which catalyzes decarboxylation of L-glutamate, and moves into a cell by a glutamate GABA antiporter to produce GABA.

In a specific example, the medium composition may include about 0.5 to 10% by weight, specifically about 1 to 7% by weight, more specifically about 1 to 5% by weight, and particularly about 1, 3, or 5% by weight of MSG with respect to a total weight of the medium composition.

The term "carbon source" as used herein refers to a substance used as a carbon energy source of a strain. As a carbon source that can be used in the present invention, commercially available carbon sources commonly used in the art can be purchased and used, and there is no particular limitation on the type thereof. For example, the carbon source may include carbohydrates such as glucose, fructose, sucrose, maltose, mannitol, sorbitol, etc.; alcohols such as sugar alcohol, glycerol, pyruvic acid, lactic acid, citric acid, etc.; and amino acids such as organic acid, glutamate, methionine, lysine, etc., but is not limited thereto. Additionally, natural organic nutrients such as starch hydrolysates, molasses, blackstrap molasses, rice bran, cassava, sugarcane residue, and corn steep liquor may be used. Specifically, carbohydrates such as glucose and sterile pretreated molasses (i.e., molasses converted to reduced sugar), etc. may be used. In addition, suitable amounts of other carbon sources may be used without limitation. These carbon sources may be used alone or in combination of two or more, but are not limited thereto.

The term "nitrogen source" as used herein means a substance used as a nitrogen energy source of a strain, which prevents damage to mycobiont caused by post-fermentation. Strains living under fermentation conditions without an energy source produces organic acid, which causes a decrease in pH and induces the death of the strain. Therefore, since the nitrogen energy source prevents the production of organic acid and the resulting decrease in pH, the death of strains may be prevented.

As a nitrogen source that can be used in the present invention, commercially available carbon sources commonly used in the art can be purchased and used, and there is no particular limitation on the type thereof. Specifically, the nitrogen source may be nonfat dry milk (skim milk), whey protein, yeast extract, malt extract, beef extract, casein hydrolysate, tryptone, cysteine, and peptone, but is not particularly limited thereto. Specifically, examples of the peptone may include soy peptone, fish peptone, proteose peptone, casein peptone, and peptone No. 3, and more specifically the peptone may be soy peptone.

In a specific example, the carbon source may be at least one selected from a group consisting of glucose, fructose, sucrose, maltose, mannitol, sorbitol, sugar alcohol, glycerol, pyruvic acid, lactic acid, citric acid, organic acid, glutamate, methionine, and lysine.

Additionally, the nitrogen source may be at least one selected from a group consisting of skim milk, whey protein, yeast extract, malt extract, beef extract, casein hydrolysate, tryptone, cysteine, and peptone.

In a specific example, the carbon source is glucose, and the nitrogen source is skim milk and soy peptone.

In a specific example, the medium composition may include about 0.5 to 5% by weight, specifically about 1 to 5% by weight, more specifically about 1 to 3% by weight, and particularly about 1, 2, or 3% by weight of a carbon source and a nitrogen source, respectively with respect to a total weight of the medium composition.

In a specific example, the medium composition includes about 1 or 2% by weight of glucose as the carbon source, and about 1% by weight of skim milk and soy peptone, respectively, as the nitrogen source.

In a specific example, Lactiplantibacillus plantarum strain may be Lactiplantibacillus plantarum BNT_G401 strain deposited as Accession No. KCCM 13179P. The Lactiplantibacillus plantarum strain may be inoculated in an amount of about 5 to 15% by weight, specifically about 5 to 10% by weight or about 10 to 15% by weight, and particularly about 10% by weight with respect to a total weight of the medium composition.

In a specific embodiment, the fermented product of the present invention, with respect to a total weight of the medium composition, is produced by inoculating a medium composition comprising about 10 to 50% by weight of Curcuma longa L. extract having a solid content of about 0.5 to 10%; about 0.5 to 10% by weight of monosodium glutamate (MSG); a carbon source, for example, about 0.5 to 5% by weight of glucose; and a nitrogen source, for example, about 0.5 to 5% by weight of soy peptone and skim milk, respectively, with 5 to 15% by weight of Lactiplantibacillus plantarum strain, for example, Lactiplantibacillus plantarum BNT_G401 strain deposited as Accession No. KCCM 13179P and fermenting the same.

In another specific embodiment, the fermented product of the present invention, with respect to a total weight of the medium composition, is produced by inoculating a medium composition comprising about 20% by weight of Curcuma longa L. extract having a solid content of about 1 to 5%; about 1 to 5% by weight, for example, about 1, 3, or 5% by weight of monosodium glutamate (MSG); a carbon source, for example, about 1 to 2% by weight, for example, about 1 or 2% by weight of glucose; a nitrogen source, for example, about 1% by weight of soy peptone; and a nitrogen source, for example, about 1% by weight of skim milk, with 10% by weight of Lactiplantibacillus plantarum strain, for example, Lactiplantibacillus plantarum BNT_G401 strain deposited as Accession No. KCCM 13179P and fermenting the same.

In another embodiment, the present invention provides a medium composition comprising a Curcuma longa L. extract having a solid content of about 0.5 to 10%; monosodium glutamate (MSG); a carbon source; and a nitrogen source. The medium composition may be used as a medium for producing GABA by inoculating the medium composition with Lactiplantibacillus plantarum strain.

The specific types and contents of each ingredient included in the medium composition are as mentioned above.

## Use of fermented product for weight loss or hangover relief

The GABA containing fermented product of the present invention may be used for weight loss or hangover relief. Specifically, when the GABA containing fermented product of the present invention was administered together with a high fat diet, it was confirmed that body weight decreased, and particularly fat mass decreased. Additionally, the weight of fatty tissues decreased as well.

Acute and chronic consumption of alcohol causes damage to various organs such as liver, kidney, brain, etc.,

and particularly induces alcoholic hepatitis and cirrhosis, resulting in death. Alcohol is rapidly metabolized to acetaldehyde by alcohol dehydrogenase (ADH), microsomal ethanol oxidizing system (MEOD) and perocidase in the liver, and it is known that the effect of acetaldehyde, an alcohol metabolite, is greater than that of alcohol in the liver.

When a small amount of alcohol is consumed under normal conditions, the alcohol that enters the liver is oxidized to acetaldehyde using alcohol dehydrogenase (ADH) in cytosol, and acetaldehyde is converted back to acetate by the action of aldehyde dehydrogenase (ALDH) in the liver cell, and decomposed into water and carbonic acid gas. Accordingly, in order to confirm the effect of relieving hangovers, it is necessary to confirm the activity of aldehyde dehydrogenase (ALDH) together with alcohol dehydrogenase (ADH).

The GABA containing fermented product of the present invention increases the activity of alcohol dehydrogenase (ADH), and shows excellent alcohol degrading ability. Additionally, the GABA containing fermented product of the present invention increases the activity of aldehyde dehydrogenase (ALDH) as well. As a result, since the GABA containing fermented product of the present invention increases the activities of ADH and ALDH at the same time, it may be effectively used for relieving hangovers.

Accordingly, the present invention provides a composition comprising the GABA containing fermented product. The composition may be used for weight loss or hangover relief.

The term "composition" as used herein includes a product comprising a specific ingredient in a specific amount, and any product that results directly or indirectly from the combination of specific ingredients in specific amounts. When the composition is related to a pharmaceutical composition, such term is intended to include a product comprising an active ingredient and an inactive ingredient constituting a carrier, and any product that results directly or indirectly from the combination, complexation or aggregation of any two or more ingredients or the dissociation of one or more ingredients, or other kind of reaction or interaction.

As used herein, "composition" includes a pharmaceutical composition to be used as medical supplies for humans, a veterinary composition to be used as medical supplies for animals, dietetic products or food for humans or animals (for example, functional food composition, health functional food, i.e., food, beverage, feed or companion animal food, or food, beverage, animal feed or companion animal food supplement), etc. Accordingly, as used herein, "composition" is used to include "pharmaceutical composition," "veterinary composition," or "food composition." The composition may further include a pharmaceutically acceptable excipient, a veterinary acceptable excipient, a food acceptable excipient, etc. according to each use.

The terms "pharmaceutically acceptable," "veterinary acceptable," or "food acceptable" as used herein refer to compounds, substances, compositions, carriers, and/or administration forms suitable to be used in contact with human or animal tissues without excessive toxicity, stimulation, allergic reaction, or other problems or complications, within the scope of sound medical or food judgment, commensurate with a reasonable benefit/risk ratio.

The terms "pharmaceutically acceptable excipient," "veterinary acceptable excipient," or "food acceptable excipient" as used herein generally mean excipients useful in producing pharmaceutical compositions which are safe, non-toxic, and biologically or otherwise undesirable, and include excipients which are acceptable for human pharmaceutical use or animal veterinary use, and even for food use. The terms "pharmaceutically acceptable excipient," "veterinary acceptable excipient," or "food acceptable ingredient" as used in the specification and claims include both one type of excipient and more than one type of such excipient. For example, the pharmaceutically, veterinary, and food acceptable excipients used in the formulation of the present invention may be diluents or inert carriers, lubricants, binders, or a combination thereof. The excipients used in the formulation of the present invention may further include fillers, antimicrobial agents, antioxidants, anticaking agents, coating agents, or a mixture thereof. Other pharmaceutically, veterinary, or food acceptable excipients may be used without any limitation.

In a specific example, the composition according to the present invention may be a therapeutic pharmaceutical or a veterinary composition for weight loss.

The composition according to the present invention may be administered to humans in various ways, and may also be administered to other mammals. Here, other mammals may be domestic animals and pets such as dogs, cats, rabbits, pigs, sheep, goats, milk cows, horses, cows, etc., but are not limited thereto.

When the composition is a pharmaceutical or veterinary composition, the composition may be manufactured in various forms for parenteral or oral administration. Representative formulations for parenteral administration include formulations for injection. In this case, preferably, the form of the composition is an isotonic aqueous solution or a suspension. Formulations for injection may be manufactured according to techniques well known in the art using suitable dispersants or wetting agents, and suspending agents. For example, each ingredient may be dissolved in a saline solution or a buffer solution to be formulated for injection. Oral formulations may include powders, granules, tablets, pills, capsules, etc., but are not limited thereto.

The composition according to the present invention may be manufactured in various formulations using drug carriers. A drug delivery system may be liposome, ethosome, elastic ethosome, targeting liposome, micro-particles, micro-capsules, nano-particles, nano-capsules, nano-fibers, etc., but is not limited thereto.

In another specific example, the composition according to the present invention may be a food composition for weight loss or hangover relief, for example, a composition to be used as a health functional food or animal feed product.

When the composition is a food composition, it may be utilized as food for weight loss, fat mass reduction, or hangover relief, for example, as the main ingredient of food, supplementary ingredient of food, food additive, functional food, beverage, etc. Food compositions may be utilized as health functional food, vitamin complexes, gum, beverages, various food, tea, various processed meat products, fish products, tofu, jellied food, noodles, bread, health supplement food, seasoning food, sauces, confectionery, candies, dairy products, other processed food, fermented food, natural seasoning, etc., but are not limited thereto.

The term "health functional food" as used herein refers to food manufactured and processed using raw materials or ingredients having functionality useful for humans. The term "functionality" means obtaining effects useful for health purposes such as regulating nutrients for the structure and function of the human body or physiological effects, etc. The health functional food of the present invention may be manufactured by a method commonly used in the art, and may be manufactured by adding raw materials and ingredients commonly added in the art during manufacture. Additionally, as far as the formulation of the health functional food is recognized as a health functional food, it may be manufactured without limitation.

In another embodiment, the present invention provides a food additive composition comprising the fermented product as an active ingredient.

The term "food additive" as used herein may mean a substance used in food for the purpose of improving preservation, improving quality, enhancing nutrition, improving flavor and appearance, etc. by being added, mixed, permeated, etc. to food when manufacturing, processing or preserving the food. The food additive may add the active ingredient of the present invention as it is or may be used together with other food or food ingredients, and the mixed amount of the active ingredient may be properly determined by a person skilled in the art according to the purpose of use.

The food additive composition comprising the fermented product of the present invention may achieve the effect of weight loss or hangover relief in addition to the food or food ingredients added.

## Method of manufacturing the fermented product

In an embodiment, the present invention provides a method for manufacturing the GABA containing fermented product, which comprises the steps of (a) dissolving in water a Curcuma longa L. extract having a solid content of 0.5 to 10%; monosodium glutamate (MSG); a carbon source; and a nitrogen source, and sterilizing the same to manufacture a medium composition; (b) inoculating the medium composition with a Lactiplantibacillus plantarum strain; and (c) fermenting the medium composition in which the strain is inoculated to manufacture a GABA containing fermented product.

Before step (a), a step of manufacturing a Curcuma longa L. extract, i.e., extracting dried Curcuma longa L. in water or ethanol to manufacture a Curcuma longa L. extract having a solid content of 0.5 to 10% may be further included.

After step (c), a step of spray-drying or freeze-drying a GABA containing fermented product may be further included.

## Effect of invention

The Lactiplantibacillus plantarum BNT_G401 strain of the present invention may effectively biosynthesize GABA from monosodium glutamate (MSG) which becomes the substrate of the GABA biosynthesis. Accordingly, when fermenting a medium composition comprising a Curcuma longa L. extract using the strain, the effects of hangover relief and weight loss are excellent, and the composition may be usefully used as a food composition, etc.

## Brief description of drawings

Fig. 1 is a result illustrating the change in live bacterial count according to the fermentation time during the manufacture of GABA biosynthesis culture medium according to the solid content of Curcuma longa L. extract using the fermented product of Examples 2 to 4;

Fig. 2 is a result illustrating the change in pH according to the fermentation time during the manufacture of GABA biosynthesis culture medium according to the solid content of Curcuma longa L. extract of Examples 2 to 4;

Fig. 3 is a result illustrating the change in acidity according to the fermentation time during the manufacture of GABA biosynthesis culture medium according to the solid content of Curcuma longa L. extract of Examples 2 to 4;

Fig. 4 is a result illustrating the change in live bacterial count according to the fermentation time during the manufacture of GABA synthesis culture medium comprising the Curcuma longa L. extract according to the added amounts of MSG and glucose of Examples 5 to 9;

Fig. 5 is a result illustrating the change in pH according to the fermentation time during the manufacture of GABA synthesis culture medium comprising the Curcuma longa L. extract according to the added amounts of MSG and glucose of Examples 5 to 9;

Fig. 6 is a result illustrating the change in acidity according to the fermentation time during the manufacture of GABA synthesis culture medium comprising the Curcuma longa L. extract according to the added amounts of MSG and glucose of Examples 5 to 9;

Fig. 7 is a result illustrating the change in MSG and GABA contents according to the fermentation time through a TLC qualitative analysis during the manufacture of GABA biosynthesis culture medium comprising the Curcuma longa L. extract of Examples 2 to 9;

Fig. 8 is a result illustrating the change in MSG and GABA contents according to the fermentation time of Example 2 through a HPLC qualitative analysis;

Fig. 9 is a result illustrating the change in size and weight according to a diet period of mice induced to obesity by high fat diet according to a dietary concentration of lyophilisate of Example 2 (Experimental Example 2);

Fig. 10 is a result analyzing the change in volume of subcutaneous fat and visceral fat through dual energy X-ray absorptiometry (DXA) to show the efficacy of fat mass reduction of mice induced to obesity by high fat diet according to a dietary concentration of lyophilisate of Example 2 (Experimental Example 2);

Fig. 11 is a result illustrating the change in fatty tissue weight and epididymal fat size of mice induced to obesity by high fat diet according to a dietary concentration of lyophilisate of Example 2 (Experimental Example 2);

Fig. 12 is a result illustrating a comparison of ADH activity measurement results of Example 2 (Experimental Example 2); and

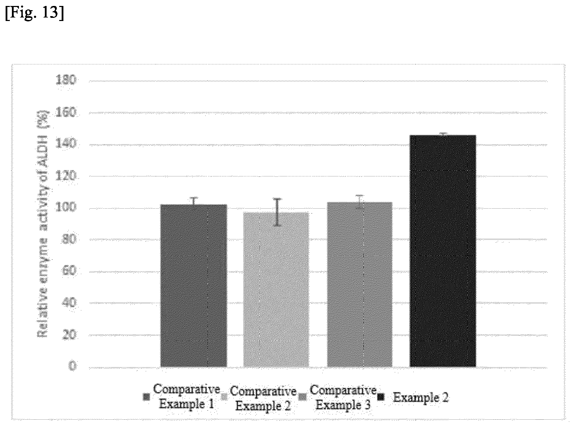

Fig. 13 is a result illustrating a comparison of ALDH activity measurement results of Example 2 (Experimental Example 4).

**Best mode for carrying out the invention**

Hereinafter, the present invention is described in more detail with examples. However, the present invention is not limited to the following examples.

**Example 1. Isolation and identification of novel lactic acid bacterium Lactiplantibacillus plantarum BNT G401**

In order to discover novel lactic acid bacterium capable of biosynthesizing GAMMA AMINOBUTYRIC ACID (GABA), isolation was carried out from fermented Kimchi. After finely chopping Kimchi, the Kimchi was diluted in sterilized purified water at a ratio of 1:9 to have a sample. The sample was diluted in purified water to $10^{-1}$ to $10^{-5}$, dispensed in 100 µl of MRS agar medium and smeared, and cultured in an incubator at 37°C for 24-48 hours to obtain a single colony. After confirming the morphological properties of the single colony, a colony presumed to be the lactic acid bacterium was fractioned and smeared on MRS agar medium, and pure isolation of the single colony was carried out two to three times.

In order to confirm GABA biosynthesis ability of the strain obtained by pure isolation, 1 loop of the strain was inoculated into MRS broth medium to which 1-3% (w/v) of monosodium glutamate (MSG) was added, and then cultured in an incubator at 37°C for 24-120 hours.

Next, the samples were collected at regular intervals and confirmed that the strain has GABA biosynthesis ability by confirming converted into biosynthetic GABA with MSG as a substrate at a spot through thin layer chromatography (TLC).

Through 16S rRNA sequencing which is a commonly known identification method, the finally selected strain was identified as Lactiplantibacillus plantarum, and the inventor of the present invention named the strain "Lactiplantibacillus plantarum BNT_G401" and deposited the same with Korean Culture Center of Microorganisms (KCCM) as Accession No. KCCM 13179P.

**Example 2. GABA biosynthetic fermented product comprising a Curcuma longa L. extract utilizing novel lactic acid bacterium, Lactiplantibacillus plantarum BNT G401**

The dried Curcuma longa L. used in the present invention was purchased at Woorinongsan and was used after washing three times in purified water. Purified water equivalent to 20 times the weight (w/w) of Curcuma longa L. was added to an ultra-high-speed vacuum low temperature concentration extractor (Kyungseo machine cosmos-700, Korea) and then extracted at 100°C for 4 hours. After the extraction, the original Curcuma longa L. was removed from the concentration extractor, and all impurities were filtered using filter paper (Whatman No. 2 Tokyo, Japan). In the present invention, Curcuma longa L. having a final solid content of 1% was used.

The medium composition for optimizing GABA biosynthesis comprising a Curcuma longa L. extract is as shown in Table 1 below. 20% by weight of the manufactured 1% Curcuma longa L. extract, 3% by weight of MSG which is the substrate for GABA biosynthesis, 1% by weight of glucose, skim milk, and soy peptone, respectively as carbon and nitrogen nutrients were mixed into purified water, and then sterilized with an autoclave sterilizer at 121°C for 15 minutes to be used as the culture medium. The culture medium was inoculated with 10% by weight of Lactiplantibacillus plantarum BNT_G401 strain starter of Example 1, and then statically cultured in an incubator at 37°C for 3 days to manufacture a fermented product. A fermented product spray-dried or freeze-dried as needed was used as a sample in the following experimental examples.

[Table 1]

| Medium composition for GABA biosynthesis of Examples 2 to 9 | | | | | | | | |
|---|---|---|---|---|---|---|---|---|
| (unit: % by weight) | Example | | | | | | | |
|  | 2 | 3 | 4 | 5 | 6 | 7 | 8 | 9 |
| 1% of Curcuma longa L. extract | 20 |  |  | 20 | 20 | 20 | 20 | 20 |
| 3% of Curcuma longa L. extract |  | 20 |  |  |  |  |  |  |
| 5% of Curcuma longa L. extract |  |  | 20 |  |  |  |  |  |
| MSG | 3 | 3 | 3 | 1 | 1 | 3 | 5 | 5 |
| Glucose | 1 | 1 | 1 | 1 | 2 | 2 | 1 | 2 |
| Soy peptone | 1 | 1 | 1 | 1 | 1 | 1 | 1 | 1 |
| Skim milk | 1 | 1 | 1 | 1 | 1 | 1 | 1 | 1 |
| Strain starter | 10 | 10 | 10 | 10 | 10 | 10 | 10 | 10 |
| D.W. | up to 100% by weight | | | | | | | |

### Example 3

The fermented product was manufactured in the same manner as in Example 2 except that in the step of manufacturing the fermented product in Example 2, the solid content of Curcuma longa L. extract is 3%.

### Example 4

The fermented product was manufactured in the same manner as in Example 2 except that in the step of manufacturing the fermented product in Example 2, the solid content of Curcuma longa L. extract is 5%.

### Example 5

The fermented product was manufactured in the same manner as in Example 2 except that in the step of manufacturing the fermented product in Example 2, MSG which is the substrate for GABA biosynthesis is used in an amount of 1% by weight.

### Example 6

The fermented product was manufactured in the same manner as in Example 2 except that in the step of manufacturing the fermented product in Example 2, MSG which is the substrate for GABA biosynthesis is used in an amount of 1% by weight, and glucose which is used as the carbon source is used in an amount of 2% by weight.

### Example 7

The fermented product was manufactured in the same manner as in Example 2 except that in the step of manufacturing the fermented product in Example 2, glucose which is used as the carbon source is used in an amount of 2% by weight.

### Example 8

The fermented product was manufactured in the same manner as in Example 2 except that in the step of manufacturing the fermented product in Example 2, MSG which is the substrate for GABA biosynthesis is used in an amount of 5% by weight.

**Example 9**

The fermented product was manufactured in the same manner as in Example 2 except that in the step of manufacturing the fermented product in Example 2, MSG which is the substrate for GABA biosynthesis is used in an amount of 5% by weight, and glucose which is used as the carbon source is used in an amount of 2% by weight.

**Comparative Example 1**

According to Table 2, 1% of Curcuma longa L. extract in Example 2, which was diluted with purified water so as to have the same concentration as the culture medium, was used.

**Comparative Example 2**

The Curcuma longa L. extract was not included in the step of manufacturing the fermented product in Example 2, and the other manufacturing steps were performed in the same manner as in Example 2 to manufacture the fermented product.

**Comparative Example 3**

Finally, 1% of Curcuma longa L. extract to same as the raw material concentration of Example 2 and fermented product of Comparative Example 2 which do not include Curcuma longa L. extract were manufactured respectively, and then mixed to be used.

In other words, except for 1% of Curcuma longa L. extract, 3% by weight of the MSG which is the substrate for GABA biosynthesis, and 1% by weight of glucose, skim milk and soy peptone, respectively as carbon and nitrogen nutrients were mixed in purified water, and then sterilized with an autoclave sterilizer at 121°C for 15 minutes to be used as the culture medium. The culture medium was inoculated with 10% by weight of Lactiplantibacillus plantarum BNT_G401 strain starter of Example 1, and then statically cultured in an incubator at 37°C for 3 days to manufacture a fermented product. Then, 20% by weight of 1% of Curcuma longa L. extract was added to the fermented product and mixed.

A fermented product spray-dried or freeze-dried as needed was used as a sample in the following experimental examples.

[Table 2]

| Medium composition for GABA biosynthesis of comparative Examples 1 to 3 | | | |
|---|---|---|---|
| (unit: % by weight) | Comparative Example | | |
| | 1 | 2 | 3 |
| 1% of Curcuma Longa L. extract | 20 | - | 20* |
| MSG | - | 3 | 3 |
| Glucose | - | 1 | 1 |
| Soy peptone | - | 1 | 1 |
| Skim milk | - | 1 | 1 |
| Strain starter | - | 10 | 10 |
| D. W. | up to 100% by weight | | |
| * Curcuma longa L. extract is additionally mixed after fermentation | | | |

**Experimental Example 1: Qualitative analysis of pH, acidity, live bacterial count, GABA content in Curcuma longa L. fermented product**

The fermented product of the Curcuma longa L. extract produced in Examples 2 to 9 was analyzed.

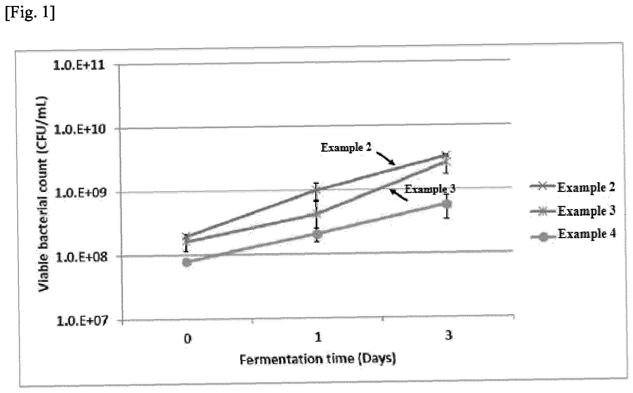

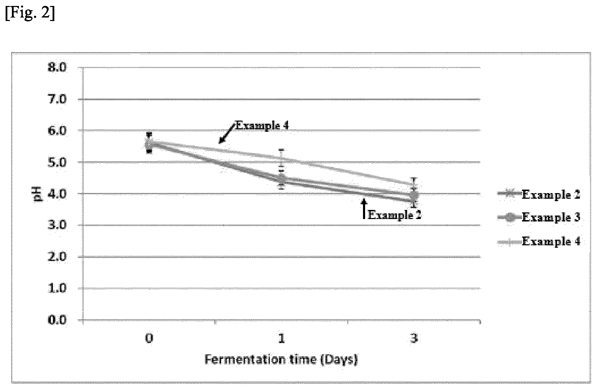

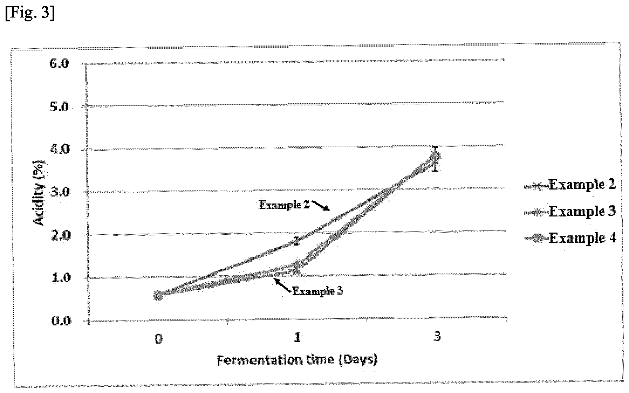

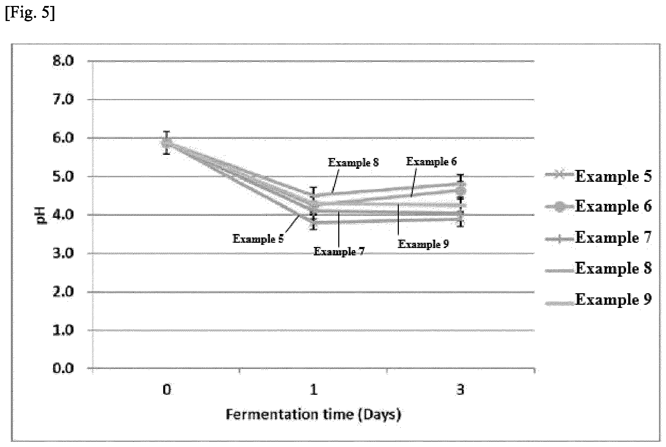

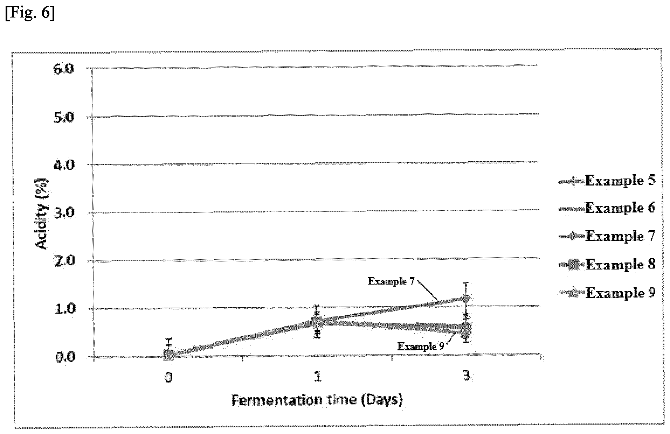

The fermented product was analyzed to confirm pH, acidity, live bacterial count, and GABA content by a TLC qualitative analysis after collecting samples at 24-hour intervals according to the culturing period. The results were shown in Figures 1 to 7.

Figs. 1 to 3 show the change in live bacterial count, pH, and acidity according to the fermentation time during the manufacture of GABA biosynthesis culture medium according to the solid content of Curcuma longa L. extract using the fermented products of Examples 2 to 4. Regardless of the solid content of Curcuma longa L. extract, a similar trend was observed during the culturing period.

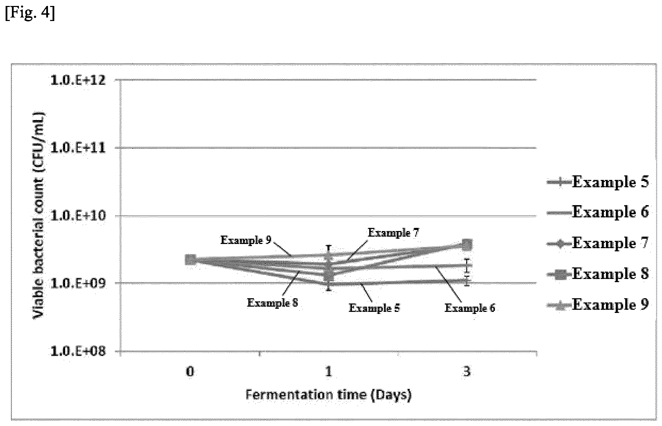

Figs. 4 to 6 show the change in live bacterial count, pH, and acidity according to the fermentation time during the manufacture of GABA biosynthesis culture medium according to the added amounts of MSG and glucose using the fermented products of Examples 5 to 9. There is a slight difference according to the added amounts of MSG and glucose, but a similar trend was observed during the culturing period.

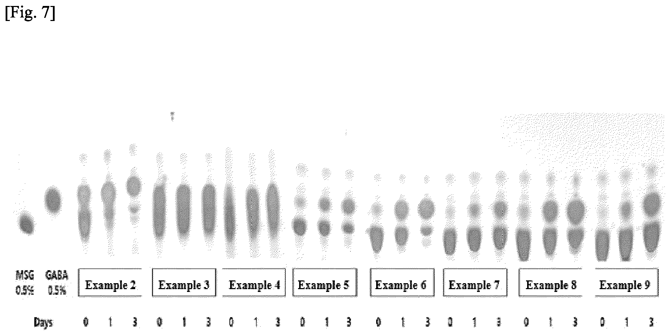

Fig. 7 confirmed the change in contents of MSG and GABA according to the fermentation time through a TLC qualitative analysis during the manufacture of GABA biosynthesis culture medium comprising the Curcuma longa L. extract in Examples 2 to 9. GABA biosynthesis was confirmed in all Examples, but considering the contents of the residual MSG and biosynthesized GABA, Example 2 showing a significant decrease in MSG content over time and GABA biosynthesis was determined to be the most suitable.

**Experimental Example 2: Quantitative analysis of MSG and GABA in Curcuma longa L. fermented product using Lactiplantibacillus plantarum BNT G401 strain**

1. Preparation of standard solution

A stock solution was prepared to prepare a standard solution for each concentration in order to analyze the contents of MSG and GABA. Specifically, using purified water with HPLC grade, for MSG, 1 mL of purified water was added to 40 mg of standard reagent to manufacture a 40,000 ppm solution, and for GABA, 1 mL of purified water was added to 20 mg of standard reagent to manufacture a 20,000 ppm solution.

Using the prepared stock solution and purified water, as for GABA, a standard solution for a calibration curve was manufactured with 13 concentration points within the 20,000-500 ppm section, and as for MSG, a standard solution for a calibration curve was manufactured with 11 concentration points within the 30,000-500 ppm section.

The standard solution manufactured was analyzed after mixing 100 $\mu$L of 1M HCl solution with 900 $\mu$L of standard solution for the same pretreatment as the analytic sample.

It was confirmed that the prepared calibration curve had a reliability of 0.9985 for MSG and 0.9895 for GABA within the concentration range. As a result of evaluating the linearity of the calibration curve, it was confirmed to have linearity with an average recovery rate of 2.31% for MSG and 3.82% for GABA.

[Table 3]

| Verification of calibration curve per ingredient | | |
| --- | --- | --- |
| Item | MSG | GABA |
| Reliability ($R^2$) | 0.9985 | 0.9895 |
| Linearity (%) | 2.31 | 3.82 |

2. Pretreatment of analytic sample

Analytic samples were collected per fermentation condition and fermentation time, and centrifuged in a centrifuge with 10,000 rpm at 4°C for 10 minutes. Next, 100 $\mu$L of 1M HCl solution was mixed with 900 $\mu$L of the supernatant, and then filtered through a 0.22 $\mu$L syringe filter to make an analytic sample.

3. HPLC condition

The HPLC-UV/vis configuration used in the present experiment used Shimadzu's SPD-40 as a UV-Vis detector.

Mobile phase A is ACN, mobile phase B is 10 mM of phosphoric buffer with pH 2.4, and mobile phase C is methanol.

Initially, mobile phases A, B and C were flown in a volume ratio of 1.5%, 97%, and 1.5%, respectively, at a flow rate of 1 mL/min. The concentration gradient of the mobile phases was linear with 45% for mobile phase A, 10% for mobile phase B, and 45% for mobile phase C at 25 minutes, and 1.5% for mobile phase A, 97% for mobile phase B, and 1.5% for mobile phase C at 29 minutes. The final composition was maintained for up to 35 minutes.

[Table 4]

| Mobile phase concentration gradient | | | |
|---|---|---|---|
| Time | A (%) | B (%) | C (%) |
| 0 | 1.5 | 97 | 1.5 |
| 25 | 45 | 10 | 45 |
| 29 | 1.5 | 97 | 1.5 |
| 35 | 1.5 | 97 | 1.5 |

As column, a 5 μm column of CAPCELL PAK C18 UG 120 (4.6x250 mm) was used. As HPLC instrument conditions, the column temperature was 40°C, the detection wavelength of the detector was 338 nm, the cell temperature was 40°C, and the detection polarity of the UV detector was positive.

The sample was derivatized and injected using an autosampler. For the derivatization process, 0.1 μL of sample, 0.2 μL of OPA reagent (o-phthalaldehyde, 3-mercaptopropionic acid in borate buffer, Agilent PIN 5061-3335), 0.5 μL of 0.4 M Borate buffer with pH 10.2, and 9.3 μL of purified water were mixed in order. Each sample was mixed three times, reacted for 30 seconds, and then injected.

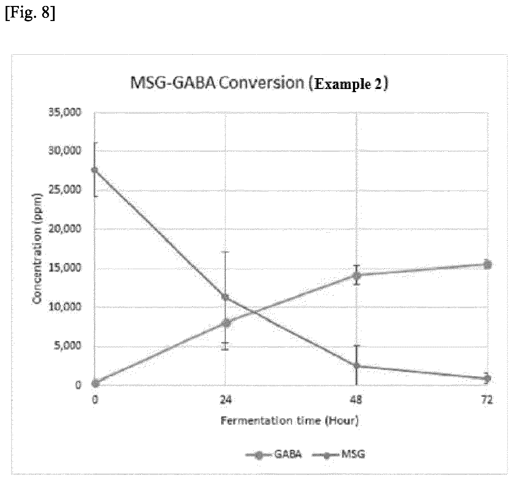

As a result, the change in MSG and GABA contents according to the culturing period of the Curcuma longa L. extract fermented product in Example 2 was shown in Fig. 8. It was confirmed that the final GABA content in Example 2 was 16,194 ppm, and the residual MSG content was 1,579 ppm.

**Experimental Example 2. Effect of reducing fat mass by Curcuma longa L. fermented product in overweight animal models**

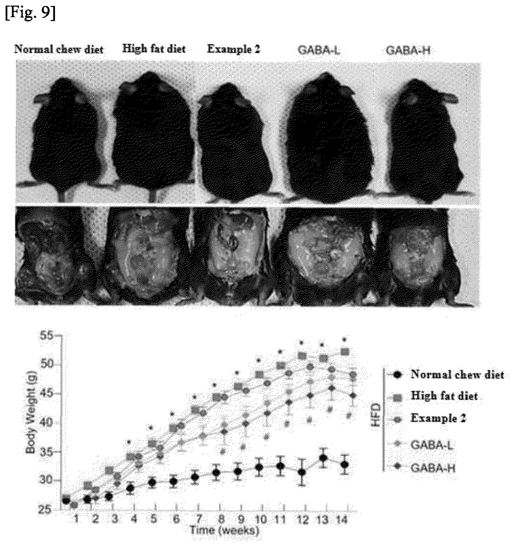

Experimental samples were spray-dried. A 7-week-old male C57BL6J mouse was purchased from Orient and adapted for a week. Then, a high-fat powder feed (60% high fat diet) was fed for 14 weeks, and Example 2 was orally administered at an administration concentration of 2 g/kg once a day for 14 weeks at a regular time. In this case, the GABA content in the administration group of Example 2 is about 0.17 g/kg. In this case, only the same amount of high fat diet was orally administered to the control group. Also, as a positive control group, high content of GABA of 2 g/kg (GABA-H) and low content of GABA of 1 g/kg (GABA-L) were orally administered once a day for 14 weeks at a regular time. The dietary amount and weight of the mouse administered with the sample were measured every week, and the result of observing the change in weight of the experimental animal was shown in Fig. 9. It was confirmed that compared to the normal chew diet, the weight was significantly increased in the high fat diet, and the weight change in the high fat diet and Example 2 co-administration group significantly decreased compared to the high fat diet group.

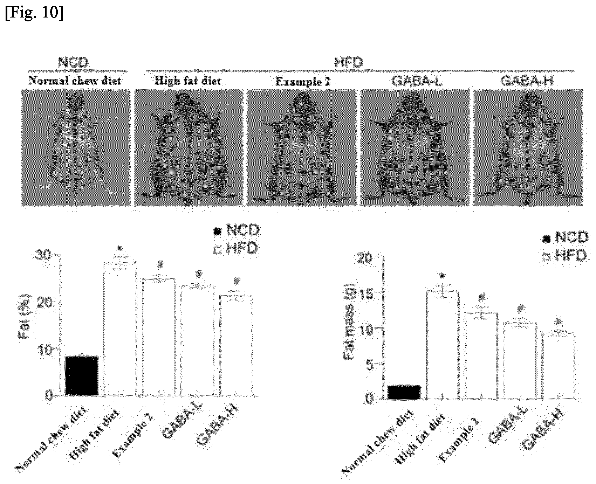

In order to figure out the effect of Example 2 on the reduction of fat mass in an overweight animal model induced by a high fat diet, the volume of subcutaneous fat and visceral fat was observed as an image by dual energy X-ray absorptiometry (DXA). As shown in Fig. 10, it was confirmed that compared to the normal chew diet (NCD), the fat mass amount significantly increased in the high fat diet (HFD), and the fat mass amount decreased in the high fat diet and Example 2 co-administration group compared to the high fat diet group.

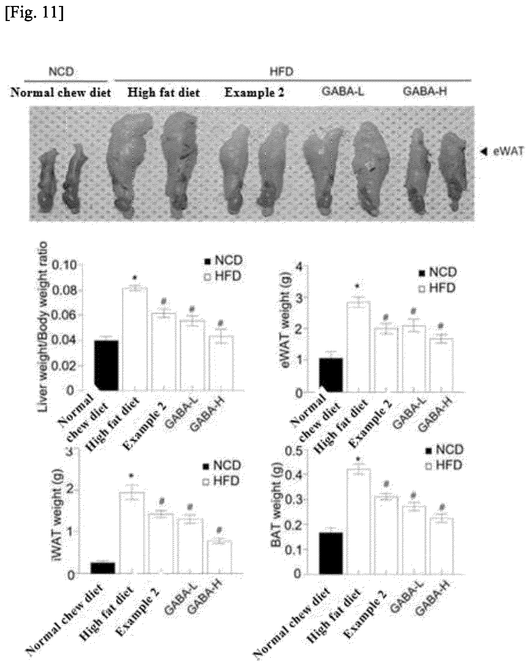

After visually inspecting all animals at the time of autopsy, the weight of the organs was measured by removing the liver, kidney, epididymal fat and mesenteric fat from the experimental animals whose abdomen was cut, washing them with a saline solution, removing moisture with filter paper, and weighing the organs. The result was shown in Fig. 11. In Fig. 11, eWAT indicates epididymal white adipose tissue, iWAT indicates inguinal white adipose tissue, and BAT indicates brown adipose tissue. It was observed that compared to the normal chew diet (NCD) group, the size of epididymal fat increased in the high fat diet (HFD) group, and it was confirmed that the size of epididymal fat significantly decreased in the high fat diet and Example 2 co-administration group compared to the high fat diet group. As a result of measuring the weight of liver, epididymal fat, abdominal fat or brown fat, it was confirmed that the weight of each tissue increased in the high fat diet group compared to the normal chew diet group, but the weight of each tissue decreased in the high fat diet and Example 2 co-administration group.

**Experimental Example 3. Experiment of alcohol dehydrogenase (ADD) activity for the Curcuma longa L. fermented product**

Experimental samples were freeze-dried. To measure ADH activity, the amount of NADH produced when reacting with ethanol was measured at an absorbance of 340 nm. First, a mixture of 0.14 mL of diluted water, 0.075 mL of 1.0 M Tris-HCl buffer solution (pH 8.8), 0.03 mL of 20 mM NAD (Sigma-Aldrich), 0.03 mL of ethanol and 0.01 mL of sample, and 0.015 mL of ADH (Sigma-Aldrich) were put into a 96 well plate to make a total of 0.3 mL. Then, after reacting at 30°C for 5 minutes, the absorbance change was measured. The ADH activity was calculated by Equation 1 below.

[Equation 1]

ADH activity (%) = (absorbance of experimental group/absorbance of control group) x

100

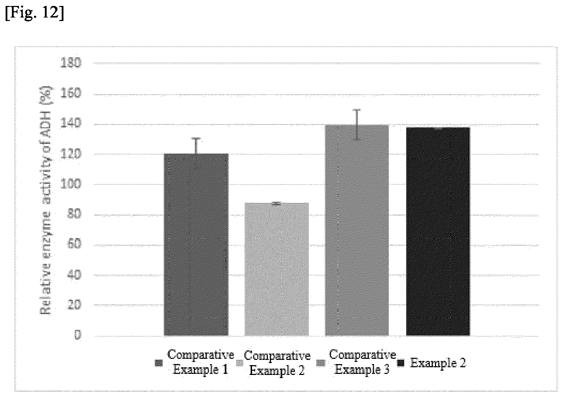

The result was shown in Fig. 12. Comparative Examples 1, 2 and 3 showed an ADH activity of 120.4%, 87.83%, and 139.4%, respectively. Example 2 showed excellent ADH activity of 137.5%.

In Comparative Example 1, only a Curcuma longa L. extract was used, so GABA biosynthesis did not proceed because there was no carbon and nitrogen source and no fermentation process by microorganisms. Example 2 in which GABA biosynthesis was confirmed through fermentation showed more excellent ADH activity compared to Comparative Example 1.

Comparative Example 2 did not include a Curcuma longa L. extract, but Example 2 showed about 50% higher ADH activity than Comparative Example 2.

Comparative Example 3 is a mixture of 1% of Curcuma longa L. extract and a fermented product without the Curcuma longa L. extract, and Example 2 showed an ADH activity almost equivalent to Comparative Example 3. This indicates that the ADH activity of Curcuma longa L. does not decrease even when it is subjected to a fermentation process with the Curcuma longa L. extract included.

Accordingly, it may be known that the Curcuma longa L. fermented product manufactured according to the present invention has excellent alcohol degrading ability because of its excellent ADH activity.

**Experimental Example 4. Experiment of aldehyde dehydrogenase (ALDH) activity for the Curcuma longa L. fermented product**

Experimental samples were freeze-dried. ALDH activity was measured using the principle of being produced NADH from NAD by an enzyme producing acetate using acetaldehyde as a substrate. First, a mixture of 0.11 mL of diluted water, 0.03 mL of 1.0 M Tris-HCl buffer solution (pH 8.0), 0.01 mL of 20 mM NAD (Sigma-Aldrich), 0.01 mL of 0.33 M Mercaptoethanol, 0.01 mL of 3.0 M KCl, 0.01 mL of 1.0 M Acetaldehyde and 0.01 mL of sample, and 0.01 mL of ALDH (Sigma-Aldrich) were put into a 96 well plate to make a total of 0.2 mL. Then, after reacting at 30°C for 5 minutes, the absorbance change was measured. The ADH activity was calculated by Equation 2 below.

[Equation 2]

ALDH activity (%) = (absorbance of experimental group/absorbance of control group) x

100

In the process of oxidizing the alcohol absorbed in the body to acetaldehyde by ADH and to acetic acid by ALDH, active oxygen ROS is formed, which causes hangovers such as headaches, nausea, etc. Thus, the activity of the ALDH enzyme is more important in relieving a hangover. As a result of the ALDH experiment, Comparative Examples 1, 2 and 3 showed an ALDH activity of 102.6%, 97.2%, and 103.8%, respectively. Example 2 manufactured according to the present invention showed the most excellent ALDH activity with an efficacy of 146.3%. It is determined that such result was made because the fermented product produced through the Curcuma longa L. fermentation process by microorganisms affects the hangover relief effect.

Although the preferred embodiments of the present invention have been described in detail above, the scope of the present invention is not limited thereto, and various modifications and improvements by those skilled in the art using the basic concept of the present invention defined in the following claims may also fall within the scope of the present invention.

[Accession No.]

Name of Deposit Authority: Korean Culture Center of Microorganisms (KCCM)
Accession No.: KCCM13179P
Date of deposition: 20220517

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. BINOTEC CO., LTD.

155, deulan-ro,

Suseong-gu,

Daegu, 42151,

Republic of Korea

RECEIPT IN THE CASE OF AN ORIGINAL DEPOSIT
issued pursuant to Rule 7.1 by the
INTERNATIONAL DEPOSITARY AUTHORITY
identified at the bottom of this page

| Ⅰ. IDENTIFICATION OF THE MICROORGANISM | |
| --- | --- |
| Identification reference given by the DEPOSITOR:<br>*Lactiplantibacillus plantarum* BNT_G401 | Accession number given by the INTERNATIONAL DEPOSITARY AUTHORITY:<br>KCCM13179P |

**Ⅱ. SCIENTIFIC DESCRIPTION AND/OR PROPOSED TAXONOMIC DESIGNATION**

The microorganism identified under I above was accompanied by:

☐ a scientific description

☐ a proposed taxonomic designation

(Mark with a cross where applicable)

**Ⅲ. RECEIPT AND ACCEPTANCE**

This International Depositary Authority accepts the microorganism identified under I above,

which was received by it on May. 17. 2022 (date of the original deposit).[1]

**Ⅳ. RECEIPT OF REQUEST FOR CONVERSION**

The microorganism identified under I above was received by this International Depositary Authority

on                         (date of the original deposit) and a request to convert the original deposit to a deposit under

the Budapest Treaty was received by it on                         (date of receipt of request for conversion).

**Ⅴ. INTERNATIONAL DEPOSITARY AUTHORITY**

| Name : Korean Culture Center of Microorganisms<br><br>Address : Yurim B/D<br>45, Hongjenae-2ga-gil<br>Seodaemun-gu<br>SEOUL 03641<br>Republic of Korea | Signature(s) of person(s) having the power to represent the International Depositary Authority or of authorized official(s):<br><br>Date: May. 17. 2022. |
| --- | --- |

[1] Where Rule 6.4(d) applies, such date is the date on which the status of international depositary authority was acquired.

Form BP/4 (sole page)

한국미생물보존센터 사단법인 한국종균협회
03641 서울특별시 서대문구 홍제내2가길 45 유림빌딩  Tel: 02-391-0950, 396-0950  Fax: 02-392-2859

KOREAN CULTURE CENTER OF MICROORGANISMS KOREAN FEDERATION OF CULTURE COLLECTIONS
Yurim Bldg., 45, Hongjenae 2ga-gil, Seodaemun-gu, Seoul, 03641, Korea.  Tel: 82-2-391-0950, 396-0950  Fax: 82-2-392-2859

**Claims**

1. A Lactiplantibacillus plantarum BNT_G401 strain deposited as Accession No. KCCM 13179P.

2. A γ-aminobutyric acid (GABA) containing fermented product, wherein the fermented product is produced by inoculating a medium composition comprising: a Curcuma longa L. extract having a solid content of 0.5 to 10%; monosodium glutamate (MSG); a carbon source; and a nitrogen source with the Lactiplantibacillus plantarum strain and fermenting the same.

3. The GABA containing fermented product of claim 2, wherein the medium composition comprises 10 to 50% by weight of Curcuma longa L. extract with respect to a total weight of the medium composition.

4. The GABA containing fermented product of claim 2, wherein the medium composition comprises 0.5 to 10% by weight of monosodium glutamate (MSG) with respect to a total weight of the medium composition.

5. The GABA containing fermented product of claim 2, wherein the carbon source is at least one selected from a group consisting of glucose, fructose, sucrose, maltose, mannitol, sorbitol, sugar alcohol, glycerol, pyruvic acid, lactic acid, citric acid, organic acid, glutamate, methionine, and lysine, and the nitrogen source is at least one selected from a group consisting of skim milk, whey protein, yeast extract, malt extract, beef extract, casein hydrolysate, tryptone, cysteine, and peptone.

6. The GABA containing fermented product of claim 2, wherein the medium composition comprises 0.5 to 5% by weight of a carbon source and a nitrogen source, respectively, with respect to a total weight of the medium composition.

7. The GABA containing fermented product of claim 2, wherein the Lactiplantibacillus plantarum strain is a Lactiplantibacillus plantarum BNT_G401 (KCCM 13179P) strain deposited as Accession No. KCCM 13179P.

8. The GABA containing fermented product of claim 2, wherein the Lactiplantibacillus plantarum strain is inoculated in an amount of 5 to 15% by weight with respect to a total weight of the medium composition.

9. The GABA containing fermented product of claim 2, wherein the fermented product, with respect to a total weight of the medium composition, is produced by inoculating a medium composition comprising 10 to 50% by weight of Curcuma longa L. extract having a solid content of 0.5 to 10%; 0.5 to 10% by weight of monosodium glutamate (MSG); 0.5 to 5% by weight of glucose as a carbon source; and 0.5 to 5% by weight of soy peptone and skim milk, respectively, as nitrogen sources with 5 to 15% by weight of Lactiplantibacillus plantarum BNT_G401 strain deposited as Accession No. KCCM 13179P.

10. A composition comprising the GABA containing fermented product according to any one of claims 1 to 9.

11. The composition of claim 10 for weight loss.

12. The composition of claim 10 for hangover relief.

13. A method for manufacturing a GABA containing fermented product according to any one of claims 1 to 9, comprising:

     (a) dissolving in water a Curcuma longa L. extract having a solid content of 0.5 to 10%; monosodium glutamate (MSG); a carbon source; and a nitrogen source, and sterilizing the same to manufacture a medium composition;
     (b) inoculating the medium composition with a Lactiplantibacillus plantarum strain; and
     (c) fermenting the medium composition in which the strain is inoculated to manufacture a GABA containing fermented product.

14. The method of claim 13, further comprising:
     extracting dried Curcuma longa L. in water or ethanol to manufacture a Curcuma longa L. extract having a solid content of 0.5 to 10%.

15. The method of claim 13, further comprising:
     spray-drying or freeze-drying a GABA containing fermented product.

[Fig. 1]

[Fig. 2]

[Fig. 3]

[Fig. 4]

[Fig. 5]

[Fig. 6]

[Fig. 7]

[Fig. 8]

MSG-GABA Conversion (Example 2)

[Fig. 9]

[Fig. 10]

[Fig. 11]

[Fig. 12]

[Fig. 13]

| INTERNATIONAL SEARCH REPORT | International application No. |
|---|---|
| | **PCT/KR2022/014864** |

**A. CLASSIFICATION OF SUBJECT MATTER**

**A23L 33/105**(2016.01)i; **A23L 29/00**(2016.01)i; **A23L 2/38**(2006.01)i; **C12N 1/20**(2006.01)i; C12R 1/01(2006.01)n

According to International Patent Classification (IPC) or to both national classification and IPC

**B. FIELDS SEARCHED**

Minimum documentation searched (classification system followed by classification symbols)

A23L 33/105(2016.01); A23L 13/60(2016.01); A23L 33/00(2016.01); C12N 1/20(2006.01); C12P 7/52(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 락티플란티바실러스(Lactiplantibacillus), 감마-아미노부티르산(gamma-amin obutyric acid), 발효(fermentation), 울금(turmeric), 글루탐산 일나트륨(monosodium glutamate), 탄소원(carbon source), 질소원(nitrogen source)

**C. DOCUMENTS CONSIDERED TO BE RELEVANT**

| Category* | Citation of document, with indication, where appropriate, of the relevant passages | Relevant to claim No. |
|---|---|---|
| X | KR 10-1480250 B1 (INDUSTRY ACADEMIC COOPERATION FOUNDATION KEIMYUNG UNIVERSITY) 09 January 2015 (2015-01-09)<br>See abstract; and claims 1-7. | 1 |
| Y | | 2-15 |
| Y | KR 10-2014-0051543 A (INDUSTRY ACADEMIC COOPERATION FOUNDATION KEIMYUNG UNIVERSITY et al.) 02 May 2014 (2014-05-02)<br>See claims 1-4; and paragraph [0026]. | 2-15 |
| Y | KR 10-2018-0132208 A (GYEONGNAM NATIONAL UNIVERSITY OF SCIENCE AND TECHNOLOGY INDUSTRY-ACADEMIC COOPERATION FOUNDATION) 12 December 2018 (2018-12-12)<br>See claims 1 and 4; and paragraph [0005]. | 11-12 |

☑ Further documents are listed in the continuation of Box C.  ☑ See patent family annex.

| | |
|---|---|
| * Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **09 January 2023** | **09 January 2023** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

International application No.

**PCT/KR2022/014864**

| C. | DOCUMENTS CONSIDERED TO BE RELEVANT |
|---|---|

| Category* | Citation of document, with indication, where appropriate, of the relevant passages | Relevant to claim No. |
|---|---|---|
| A | KR 10-2018-0012477 A (KOREA UNIVERSITY RESEARCH AND BUSINESS FOUNDATION, SEJONG CAMPUS) 06 February 2018 (2018-02-06)<br>See entire document. | 1-15 |
| A | KR 10-2012-0007917 A (CHUNG ANG UNIVERSITY INDUSTRY ACADEMIC COOPERATION FOUNDATION et al.) 25 January 2012 (2012-01-25)<br>See entire document. | 1-15 |

Form PCT/ISA/210 (second sheet) (July 2022)

**INTERNATIONAL SEARCH REPORT**
Information on patent family members

International application No.

**PCT/KR2022/014864**

| Patent document cited in search report | | | Publication date (day/month/year) | Patent family member(s) | | | Publication date (day/month/year) |
|---|---|---|---|---|---|---|---|
| KR | 10-1480250 | B1 | 09 January 2015 | None | | | |
| KR | 10-2014-0051543 | A | 02 May 2014 | KR | 10-1402031 | B1 | 02 June 2014 |
| KR | 10-2018-0132208 | A | 12 December 2018 | KR | 10-1974723 | B1 | 02 May 2019 |
| KR | 10-2018-0012477 | A | 06 February 2018 | None | | | |
| KR | 10-2012-0007917 | A | 25 January 2012 | KR | 10-1192307 | B1 | 17 October 2012 |

Form PCT/ISA/210 (patent family annex) (July 2022)

**EP 4 289 288 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020110106924
- KR 1020120066803
- KR 1020160035454